# FASCICULE DE BREVET EUROPEEN

(11) **EP 0 672 433 B1**
(45) Date de publication et mention de la délivrance du brevet: **01.06.2005**
(21) Numéro de dépôt: 95400559.1
(22) Date de dépôt: 15.03.1995
(51) Int. Cl.: A61N 1/365

(54) **Procédé de commande de la fréquence de base d'un stimulateur cardiaque**
Verfahren zur Steuerung der Grundfrequenz eines Herzschrittmachers
Method for controlling the base frequency of a pacemaker

(30) Priorité: 16.03.1994 FR 9403039
(43) Date de publication de la demande: 20.09.1995
(73) Titulaire: ELA MEDICAL, F-92541 Montrouge Cédex (FR)
(72) Inventeur: Bouhour, Anne, F-75013 Paris (FR); Bonnet, Jean-Luc, F-92170 Vanves (FR); Limousin, Marcel, F-92120 Montrouge (FR)
(74) Mandataire: Laget, Jean-Loup

(56) Documents cités:
- EP-A- 0 080 348
- EP-A- 0 203 027
- EP-A- 0 313 881
- EP-A- 0 326 629
- EP-A- 0 493 220
- WO-A-91/01157

## Description

L'invention concerne un stimulateur cardiaque et notamment un stimulateur cardiaque muni d'au moins un capteur d'un paramètre physiologique. Ce capteur assure la fonction de contrôle de la fréquence de base du stimulateur. Il peut aussi remplir la fonction d'asservissement de la fréquence de stimulation, mais cette dernière fonction peut être assurée par un autre capteur.

Dans un stimulateur cardiaque, il est usuel de définir une fréquence de base programmée, au dessous de laquelle des impulsions de stimulation sont délivrées. Toutefois, durant les périodes de repos, un rythme spontané inférieur à cette fréquence de base mais compatible avec l'état du patient, peut s'établir. Il ne pourra cependant pas s'exprimer du fait du réglage de l'appareil.

Plusieurs solutions ont été utilisées afin de faire varier la fréquence de base en fonction des périodes de repos et d'activité du patient.

Le document EP-A-0 493 220 décrit un stimulateur cardiaque à fréquence de stimulation asservie à la demande métabolique du patient. La fréquence de stimulation est adaptée en fonction du niveau d'activité du patient. Pour éviter qu'un trou ventilatoire soit perçu comme une fin d'effort et soit immédiatement suivi d'une baisse de la fréquence de stimulation, la fréquence de stimulation reste constante jusqu'à une confirmation de la fin d'effort.

Le brevet US 5 143 065 décrit un modèle prédictif, en fonction des heures du jour et de la nuit, des besoins physiologiques cycliques du patient.

Le document WO 86/07270 décrit une fonction simulant la variation circadienne du rythme cardiaque naturel. Aucun de ces documents ne permet l'adaptation de la fréquence de base aux périodes d'activité réelle ou de repos effectif du patient. Le Manuel du Praticien ("Technical Manual") Ergos TC 03, BIOTRONIK, 1993, est considéré comme l'état de la technique le plus proche.

Un but de l'invention est de proposer un stimulateur cardiaque dont la fréquence de base programmée s'adapte automatiquement aux besoins physiologiques du patient, aussi bien pendant ses phases de repos diurne ou nocturne que pendant ses phases de retour à l'activité.

La présente invention a pour objet un stimulateur cardiaque comme défini dans la revendication 1.

Selon d'autres caractéristiques :
- il analyse l'information du capteur, et le rythme cardiaque pour détecter les phases de repos et d'activité du patient ;
- il définit une phase de repos lorsqu'il détecte simultanément une phase de repos capteur et une phase de repos cardiaque ;
- il définit une phase d'activité lorsqu'il détecte soit une phase d'activité capteur, soit une phase d'activité cardiaque ;
- il définit une phase de repos capteur lorsque, r fois sur p, avec r≤p, la moyenne capteur est inférieure au niveau de repos du capteur ;
- la moyenne capteur est la moyenne des dernières informations du capteur au cours d'une première durée programmable définie soit par une horloge, soit par un nombre de cycles respiratoires ;
- il calcule la moyenne capteur à intervalles réguliers dont la durée correspond à une deuxième durée programmable définie soit par une horloge, soit par un nombre de cycles respiratoires ;
- il définit une phase de repos cardiaque lorsque pendant N cycles cardiaques, le pourcentage de cycles cardiaques pour lesquels l'intervalle entre deux événements cardiaques de même nature consécutifs est supérieur à l'intervalle de base diminué d'un seuil, est supérieur à une valeur Y% programmable,
- il définit une phase de repos cardiaque lorsque pendant S cycles cardiaques, l'intervalle entre deux évènements cardiaques de même nature consécutifs est supérieur à l'intervalle de base actuel diminué d'un seuil;
- il prolonge d'un incrément programmable l'intervalle de base actuel après chaque phase de repos;
- il prolonge d'un incrément programmable l'intervalle de base actuel jusqu'à atteindre la fréquence de base minimale;
- il diminue d'un décrément programmable l'intervalle de base actuel après chaque phase de reprise d'activité;
- il diminue d'un décrément programmable l'intervalle de base actuel jusqu'à atteindre la fréquence de base programmée ;
- il définit une extrasystolie trop importante lorsque pendant N cycles cardiaques, le nombre d'extrasystoles auriculaires ou ventriculaires est supérieur à une valeur M programmable ;
- il diminue d'un décrément l'intervalle de base actuel lorsqu'il a défini une extrasystolie trop importante;
- il définit une phase d'activité capteur lorsque, moins de r fois sur p, avec r≤p, la moyenne capteur est inférieure au niveau de repos du capteur ;
- il définit une phase d'activité cardiaque lorsque pendant N cycles cardiaques, le pourcentage de cycles cardiaques pour lesquels l'intervalle entre deux événements cardiaques de même nature consécutifs est supérieur à l'intervalle de base actuel diminué d'un seuil, est inférieur ou égal à une valeur Y% programmable,
- il définit une phase d'activité cardiaque lorsque pendant S cycles cardiaques, l'intervalle entre deux évènements cardiaques de même nature consécutifs est inférieur ou égal à l'intervalle de base actuel diminué d'un seuil;
- l'asservissement de la fréquence de stimulation à au moins un capteur reste toujours fonctionnel quelle que soit la fréquence de base actuelle.

D'autres caractéristiques ressortent de la description qui suit faite avec référence aux dessins annexés sur lesquels on peut voir :
Figure 1 : un schéma d'application du procédé de commande de la fréquence de base du stimulateur;
Figure 2 : un schéma représentatif de l'algorithme de contrôle du capteur ;
Figure 3 : un schéma représentatif de l'algorithme de mise en oeuvre du procédé de commande de la fréquence de base.

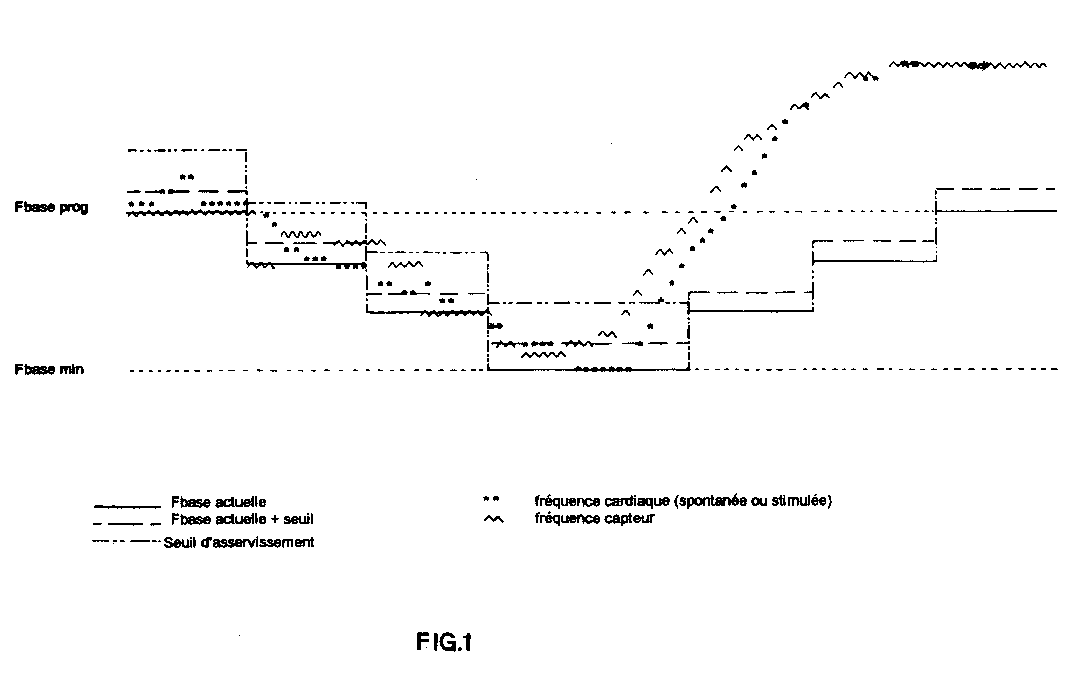

La figure 1 représente les variations, par paliers, de la fréquence de base, entre la fréquence de base programmée et la fréquence de base minimale, pendant une période de repos du patient, puis, entre la fréquence de base minimale et la fréquence de base programmée, après la reprise d'activité du porteur représentée par la montée de la fréquence capteur.

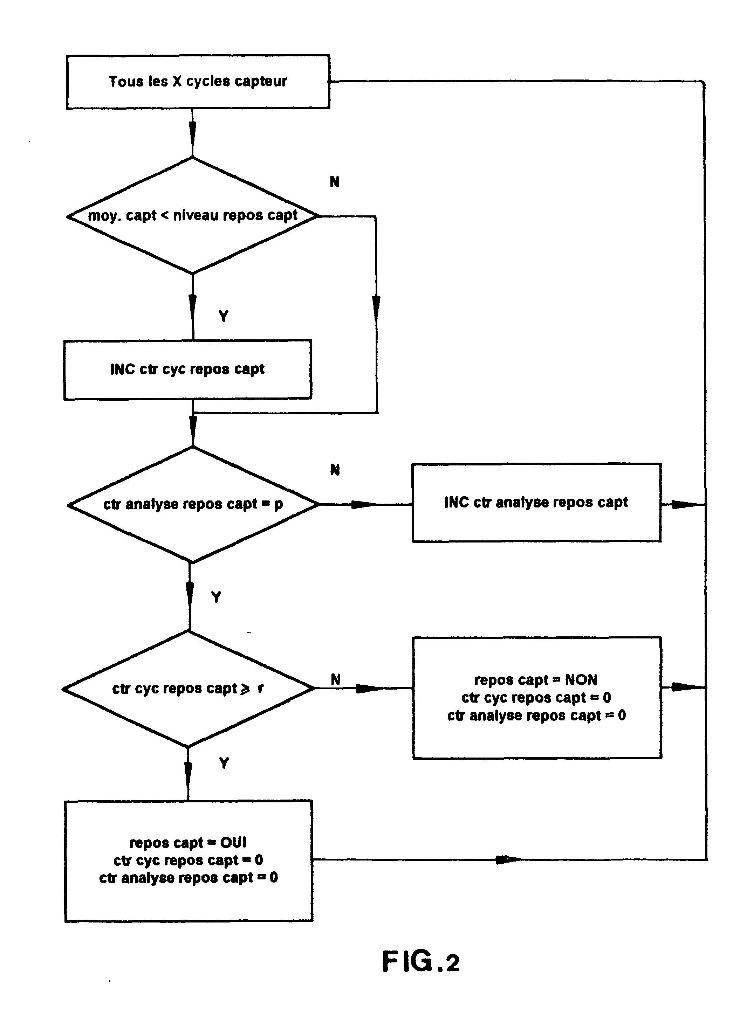

La figure 2 représente l'algorithme de contrôle du capteur, ayant pour objet de définir, par l'information fournie par le capteur, s'il y a un état de repos dénommé état de repos capteur.

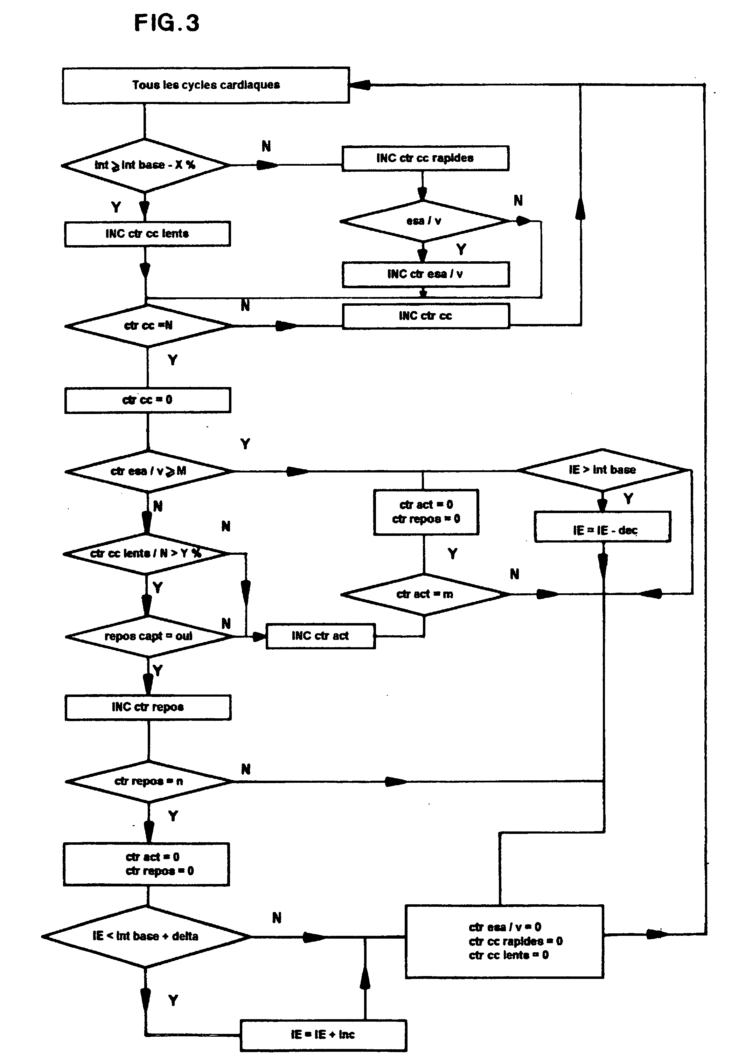

La figure 3 représente l'algorithme de contrôle du stimulateur, ayant pour objet de définir, par le suivi des événements cardiaques, s'il y a un état de repos dénommé état de repos cardiaque.

Lorsque l'état de repos est défini à la fois par l'information fournie par le capteur et par le suivi des événements cardiaques, c'est-à-dire qu'il y a à la fois un état de repos capteur et un état de repos cardiaque, cet algorithme contrôle la diminution de la fréquence de base depuis la fréquence de base programmée jusqu'à la fréquence de base minimale. Cette diminution s'effectue automatiquement et par paliers.

Lorsque pendant une période de repos, une activité est détectée, l'algorithme contrôle l'augmentation de la fréquence de base, depuis la fréquence de base minimale jusqu'à la fréquence de base programmée. Cette augmentation s'effectue automatiquement et par paliers.

L'état de repos ou d'activité du patient est détecté, par exemple, par un capteur d'activité détectant les mouvements du tronc, ou par un capteur mesurant la ventilation-minute. Plusieurs capteurs peuvent être prévus.

Pour définir un état de repos capteur, une moyenne des dernières informations du capteur est calculée à intervalles réguliers. Ces intervalles sont programmables et correspondent par exemple à 32 cycles respiratoires.

Le nombre des dernières informations du capteur est programmable et correspond par exemple à 128 cycles respiratoires. La moyenne capteur, ou moyenne des dernières informations du capteur est par exemple la moyenne de la ventilation-minute sur les 128 derniers cycles respiratoires.

Le capteur peut suivre un paramètre sans liaison avec la respiration du patient. Par exemple, il peut être du type accélérométrique pour suivre l'activité du patient. Dans ce cas, il est possible de faire référence à une durée plutôt qu'à un nombre de cycles respiratoires.

Un repos capteur, est défini lorsque la moyenne capteur est inférieure au niveau de repos du capteur, par exemple r fois sur p, avec r≤p. Les nombres r et p sont programmables. Le niveau de repos du capteur est déterminé en fonction du rythme journalier du patient, par exemple par calibration automatique selon le document FR-A-2-671 013.

Une activité capteur est définie lorsque, moins de r fois sur p, avec r≤p, la moyenne capteur est inférieure au niveau de repos du capteur.

Un état de repos cardiaque, est déterminé tous les N cycles cardiaques, le nombre N étant programmable.

Un cycle cardiaque est défini par un intervalle compris entre deux événements cardiaques de même nature consécutifs, par exemple l'intervalle compris entre deux ondes P consécutives, ou l'intervalle compris entre deux ondes R consécutives. Dans le cadre de la présente invention, l'intervalle est usuellement défini comme la durée entre deux ondes R consécutives.

Durant les N cycles cardiaques, est calculé le pourcentage de cycles cardiaques, spontanés ou stimulés, pour lesquels l'intervalle entre deux événements cardiaques de même nature consécutifs est supérieur à l'intervalle de base actuel diminué d'un seuil.

Si ce pourcentage est supérieur à une valeur programmable Y%, alors un repos cardiaque est défini.

Si ce pourcentage est inférieur ou égal à cette valeur programmable Y%, alors une activité cardiaque est définie.

En variante, il est possible de définir l'état de repos cardiaque lorsque, pendant S cycles cardiaques, l'intervalle entre deux évènements cardiaques de même nature consécutifs est supérieur à l'intervalle de base actuel diminué d'un seuil. L'état d'activité cardiaque est alors défini lorsque, pendant S cycles cardiaques, ledit intervalle est inférieur ou égal à l'intervalle de base actuel diminué d'un seuil.

Sur la figure 3 on appelle cc rapides les cycles cardiaques dont la fréquence est supérieure à la fréquence de base actuelle augmentée d'un seuil. On appelle cc lents les cycles cardiaques dont la fréquence est inférieure ou égale à la fréquence de base actuelle augmentée d'un seuil.

Pendant les N cycles cardiaques, le nombre d'extrasystoles auriculaires ou ventriculaires est mémorisé dans un compteur.

Si le nombre d'extrasystoles est supérieur à une valeur M programmable, alors une extrasystolie trop importante est définie.

Le processus de ralentissement ou d'accélération de la fréquence de base est contrôlé par l'algorithme de la figure 3 de la manière suivante :

Si pendant un nombre n programmable (ctr repos) de périodes de N cycles cardiaques, un repos capteur et un repos cardiaque sont simultanément définis, alors l'intervalle de base actuel est prolongé d'un incrément programmable, jusqu'à atteindre la fréquence de base minimale. Cette fréquence de base minimale est égale à la fréquence de base programmée diminuée d'un delta programmable de fréquence.

Si pendant un nombre m programmable (ctr act) de périodes de N cycles cardiaques, une activité capteur ou une activité cardiaque est définie, alors l'intervalle de base actuel est diminué d'un décrément programmable, jusqu'à atteindre la fréquence de base programmée.

Si pendant une période de N cycles cardiaques, une extrasystolie trop importante est définie, alors l'intervalle de base actuel est diminué d'un décrément programmable, jusqu'à atteindre la fréquence de base programmée.

Le stimulateur cardiaque utilisé est par exemple du type double chambre asservi. Lorsque les conditions du repos sont réunies, c'est-à-dire lorsque le stimulateur a détecté une phase de repos, l'intervalle de base actuel est augmenté d'une durée programmable, typiquement de 8 à 16 ms, jusqu'à atteindre l'intervalle de base maximal correspondant à la fréquence de base minimale.

Le patient peut revenir à une phase d'activité après une période de repos, c'est-à-dire alors que la fréquence de base actuelle est inférieure à la fréquence de base programmée.

Lorsque le stimulateur détecte une phase de reprise d'activité, l'intervalle de base actuel est diminué d'une durée programmable, typiquement de 8 à 16 ms, jusqu'à atteindre l'intervalle de base programmé correspondant à la fréquence de base programmée.

L'asservissement reste toujours fonctionnel, quelle que soit la fréquence de base actuelle, et ne modifie pas le fonctionnement de l'algorithme.

Un cycle avec extrasystole auriculaire ou ventriculaire ne modifie pas le comptage en cours des cycles de repos.

Les paramètres programmables sont par exemple les suivants, avec leur fourchette de valeurs et l'indication d'une valeur nominale :

| **Paramètre** | **Valeurs** | **Nominal** |
|---|---|---|
| Int. de calcul de la moyenne capteur | 16-128 | 32 cyc. resp. |
| | | |
| Nb de cycles de la moyenne capteur | 32-256 | 128 cyc. resp. |
| | | |
| Phase de repos capteur r et p (r≤p) | 1-10 | 1 |
| | | |
| Phase de repos cardiaque ou de reprise d'activité (Y %) | 50-100 % | 87.75% |
| | | |
| Seuil | 6.25-50 % | 25% |
| | | |
| Nb de cycles pour le repos cardiaque (N) | 10-100 | 32 cyc.card. |
| | | |
| Nb de cycles cardiaques (S) | 10-1024 | 50 |
| | | |
| Nb max d'extrasystoles au repos (M) | 0-50 | 5 |
| | | |
| Delta de fréquence de base | 0-45 | 20 coups par minute |
| Décelérationde la fréquence de base (n) | 1-10 | 3 |
| | | |
| Accélération de la fréquence de base (m) | 1-10 | 1 |

Le procédé de commande de la fréquence de base présente plusieurs avantages :

Tout d'abord, il permet l'expression du rythme spontané en dessous de la fréquence de base programmée, ce qui, pour le coeur, est préférable à un rythme stimulé.

Ensuite, et par voie de conséquence, la durée de service du stimulateur se trouve allongée.

Ainsi le procédé de commande permet d'adapter automatiquement la fréquence de base actuelle aux périodes d'activité et de repos du patient.

La description qui précède se rapporte à un mode particulier de réalisation de l'invention. Des variantes peuvent être prévues sans sortir du cadre de l'invention. Par exemple, les durées d'observation mentionnées dans la description peuvent être remplacées par des moyennes glissantes, notamment pour la phase de repos.

## Revendications

1. Stimulateur cardiaque muni d'au moins un capteur d'un paramètre physiologique, ledit stimulateur étant adapté à détecter les phases de repos et d'activité du patient pour y adapter automatiquement la fréquence de base du stimulateur et à contrôler la diminution de la fréquence de base depuis la fréquence de base programmée jusqu'à la fréquence de base minimale lorsque l'état de repos est détecté.

2. Stimulateur selon la revendication 1, **caractérisé en ce qu'**il analyse l'information du capteur et le rythme cardiaque pour détecter les phases de repos et d'activité du patient.

3. Stimulateur selon la revendication 2, **caractérisé en ce qu'**il définit une phase de repos lorsqu'il détecte simultanément une phase de repos capteur et une phase de repos cardiaque.

4. Stimulateur selon la revendication 2, **caractérisé en ce qu'**il définit une phase d'activité lorsqu'il détecte soit une phase d'activité capteur, soit une phase d'activité cardiaque.

5. Stimulateur selon la revendication 3, **caractérisé en ce qu'**il définit une phase de repos capteur lorsque r fois sur p, avec r≤p, la moyenne capteur est inférieure au niveau de repos du capteur.

6. Stimulateur selon la revendication 5, **caractérisé en ce que** la moyenne capteur est la moyenne des dernières informations du capteur au cours d'une première durée programmable définie soit par une horloge, soit par un nombre de cycles respiratoires.

7. Stimulateur selon la revendication 6, **caractérisé en ce qu'**il calcule la moyenne capteur à intervalles réguliers dont la durée correspond à une deuxième durée programmable définie soit par une horloge, soit par un nombre de cycles respiratoires.

8. Stimulateur selon la revendication 3, **caractérisé en ce qu'**il définit une phase de repos cardiaque lorsque pendant N cycles cardiaques, le pourcentage de cycles cardiaques pour lesquels l'intervalle entre deux événements cardiaques de même nature consécutifs est supérieur à l'intervalle de base diminué d'un seuil, est supérieur à une valeur Y% programmable.

9. Stimulateur selon la revendication 3, **caractérisé en ce qu'**il définit une phase de repos cardiaque lorsque pendant S cycles cardiaques, l'intervalle entre deux événements cardiaques de même nature consécutifs est supérieur à l'intervalle de base actuel diminué d'un seuil.

10. Stimulateur selon la revendication 3, **caractérisé en ce qu'**il prolonge d'un incrément programmable l'intervalle de base actuel après chaque phase de repos.

11. Stimulateur selon la revendication 10, **caractérisé en ce qu'**il prolonge d'un incrément programmable l'intervalle de base actuel jusqu'à atteindre la fréquence de base minimale.

12. Stimulateur selon la revendication 4, **caractérisé en ce qu'**il diminue d'un décrément programmable l'intervalle de base actuel après chaque phase de reprise d'activité.

13. Stimulateur selon la revendication 12 **caractérisé en ce qu'**il diminue d'un décrément programmable l'intervalle de base actuel jusqu'à atteindre la fréquence de base programmée.

14. Stimulateur selon la revendication 2, **caractérisé en ce qu'**il définit une extrasystolie trop importante lorsque pendant N cycles cardiaques, le nombre d'extrasystoles auriculaires ou ventriculaires est supérieur à une valeur M programmable.

15. Stimulateur selon la revendication 14, **caractérisé en ce qu'**il diminue d'un décrément l'intervalle de base actuel lorsqu'il a défini une extrasystolie trop importante.

16. Stimulateur selon la revendication 4, **caractérisé en ce qu'**il définit une phase d'activité capteur lorsque, moins de r fois sur p, avec r≤p, la moyenne capteur est inférieure au niveau de repos du capteur.

17. Stimulateur selon la revendication 4, **caractérisé en ce qu'**il définit une phase d'activité cardiaque lorsque, pendant N cycles cardiaques, le pourcentage de cycles cardiaques pour lesquels l'intervalle entre deux événements cardiaques de même nature consécutifs est supérieur à l'intervalle de base actuel diminué d'un seuil, est inférieur ou égal à une valeur Y% programmable.

18. Stimulateur selon la revendication 4, **caractérisé en ce qu'**il définit une phase d'activité cardiaque lorsque pendant S cycles cardiaques, l'intervalle entre deux évènements cardiaques de même nature consécutifs est inférieur ou égal à l'intervalle de base actuel diminué d'un seuil.

19. Stimulateur selon la revendication 1, **caractérisé en ce que** l'asservissement de la fréquence de stimulation à au moins un capteur reste toujours fonctionnel quelle que soit la fréquence de base actuelle.

## Patentansprüche

1. Herzschrittmacher, der mit mindestens einem Sensor zum Erfassen eines physiologischen Parameters ausgestattet ist, wobei der Herzschrittmacher zur Feststellung der Ruhe - und Aktivitätsphasen des Patienten abgepasst ist, um die Grundfrequenz des Herzschrittmacher automatisch an diese anzupassen und die Abnahme der Grundfrequenz von der programmierten Grundfrequenz bis zur minimalen Grundfrequenz zu steuern, wenn der Ruhezustand festgestellt ist.

2. Schrittmacher nach Anspruch 1, **dadurch gekennzeichnet, dass** er die Informationen des Sensors sowie den Herzrhythmus analysiert, um die Ruhe- und Aktivitätsphasen des Patienten festzustellen.

3. Schrittmacher nach Anspruch 2, **dadurch gekennzeichnet, dass** er es als Ruhephase definiert, wenn er zur gleichen Zeit eine Ruhephase des Sensors und eine Herzruhephase feststellt.

4. Schrittmacher nach Anspruch 2, **dadurch gekennzeichnet, dass** er es als Aktivitätsphase definiert, wenn er entweder eine Aktivitätsphase des Sensors oder eine Phase der Herzaktivität feststellt.

5. Schrittmacher nach Anspruch 3, **dadurch gekennzeichnet, dass** er es als Ruhephase des Sensors definiert, wenn der Sensordurchschnitt r Mal von p, wobei r ≤ p, unter dem Ruheniveau des sensors liegt.

6. Schrittmacher nach Anspruch 5, **dadurch gekennzeichnet, dass** der Sensordurchschnitt der Durchschnitt der letzten Informationen des Sensors im Verlauf eines ersten Zeitraums ist, der entweder uhrzeitlich oder durch eine Anzahl an Atemzyklen programmierbar definiert wird.

7. Schrittmacher nach Anspruch 6, **dadurch gekennzeichnet, dass** er den Sensordurchschnitt in regelmäßigen Intervallen berechnet, deren Dauer einem zweiten Zeitraum entspricht, der entweder uhrzeitlich oder durch eine Anzahl an Atemzyklen programmierbar definiert wird.

8. Schrittmacher nach Anspruch 3, **dadurch gekennzeichnet, dass** er es als Herzruhephase definiert, wenn im Verlauf von N Herzzyklen der Prozentsatz an Herzzyklen, bei denen das Intervall zwischen zwei aufeinander folgenden, gleichartigen kardialen Ereignissen über dem um einen Schwellenwert verringerten Grundintervall liegt, über einem programmierbaren Wert Y % liegt.

9. Schrittmacher nach Anspruch 3, **dadurch gekennzeichnet, dass** er es als Herzruhephase definiert, wenn im Verlauf von S Herzzyklen das Intervall zwischen zwei aufeinander folgenden, gleichartigen kardialen Ereignissen über dem um einen Schwellenwert verringerten, aktuellen Grundintervall liegt.

10. Schrittmacher nach Anspruch 3, **dadurch gekennzeichnet, dass** er nach jeder Ruhephase das aktuelle Grundintervall um ein programmierbares Inkrement verlängert.

11. Schrittmacher nach Anspruch 10, **dadurch gekennzeichnet, dass** er das aktuelle Grundintervall so lange um ein programmierbares Inkrement verlängert, bis die Mindestgrundfrequenz erreicht wird.

12. Schrittmacher nach Anspruch 4, **dadurch gekennzeichnet, dass** er das aktuelle Grundintervall nach jeder Phase der Wiederaufnahme der Aktivität um ein programmierbares Dekrement verringert.

13. Schrittmacher nach Anspruch 12, **dadurch gekennzeichnet, dass** er das aktuelle Grundintervall so lange um ein programmierbares Dekrement verringert, bis die programmierte Grundfrequenz erreicht wird.

14. Schrittmacher nach Anspruch 2, **dadurch gekennzeichnet, dass** er es als eine zu hohe Häufigkeit von Extrasystolen definiert, wenn im Verlauf von N Herzzyklen die Anzahl an aurikulären bzw. ventrikulären Extrasystolen über einem programmierbaren Wert M liegt.

15. Schrittmacher nach Anspruch 14, **dadurch gekennzeichnet, dass** er das aktuelle Grundintervall um ein Dekrement verringert, wenn er eine zu hohe Häufigkeit von Extrasystolen definiert hat.

16. Schrittmacher nach Anspruch 4, **dadurch gekennzeichnet, dass** er es als Aktivitätsphase des Sensors definiert, wenn der Sensordurchschnitt weniger als r Mal von p, wobei r ≤ p, unter dem Ruheniveau des Sensors liegt.

17. Schrittmacher nach Anspruch 4, **dadurch gekennzeichnet, dass** er es als Phase der Herzaktivität definiert, wenn im Verlauf von N Herzzyklen der Prozentsatz an Herzzyklen, bei denen das Intervall zwischen zwei aufeinander folgenden, gleichartigen kardialen Ereignissen über dem um einen Schwellenwert verminderten aktuellen Grundintervall liegt, niedriger oder gleich einem programmierbaren Wert Y % ist.

18. Schrittmacher nach Anspruch 4, **dadurch gekennzeichnet, dass** er es als Herzaktivität definiert, wenn im Verlauf von S Herzzyklen das Intervall zwischen zwei aufeinander folgenden, gleichartigen kardialen Ereignissen unter dem um einen Schwellenbetrag verminderten aktuellen Grundintervall liegt bzw. gleich diesem ist.

19. Schrittmacher nach Anspruch 1, **dadurch gekennzeichnet, dass** die Steuerung der Stimulationsfrequenz mit mindestens einem Sensor unabhängig von der aktuellen Grundfrequenz stets betriebsbereit bleibt.

## Claims

1. A cardiac pacemaker provided with at least one sensor for a physiological parameter, said pacemaker being adapted to detect the rest and activity phases of the patient in order to adapt automatically thereto the base frequency of the pacemaker and to control the diminution of the programmed base frequency down to the minimum base frequency when the rest condition is detected.

2. A pacemaker according to Claim 1, **characterised in that** it analyses the information from the sensor and the heart rate in order to detect the rest and activity phases of the patient.

3. A pacemaker according to Claim 2, **characterised In that** it defines a rest phase when it detects simultaneously a sensor rest phase and a cardiac rest phase.

4. A pacemaker according to Claim 2, **characterised in that** it defines an activity phase when it detects either a sensor activity phase or a cardiac activity phase.

5. A pacemaker according to Claim 3, **characterised in that** it defines a sensor rest phase when r times out of p, with r ≤p, the sensor average is less than the rest level of the sensor.

6. A pacemaker according to Claim 5, **characterised in that** the sensor average is the average of the last information from the sensor during a first programmable duration defined either by a clock or by a number of respiratory cycles.

7. A pacemaker according to Claim 6, **characterised in that** it calculates the sensor average at regular intervals, the duration of which corresponds to a second programmable duration defined either by a clock or by a number of respiratory cycles.

8. A pacemaker according to Claim 3, **characterised in that** it defines a cardiac rest phase when, during N cardiac cycles, the percentage of cardiac cycles for which the interval between two consecutive cardiac events of the same nature is greater than the base interval reduced by a threshold is greater than a programmable value Y%.

9. A pacemaker according to Claim 3, **characterised in that** it defines a cardiac rest phase when during S cardiac cycles, the interval between two consecutive cardiac events of the same nature is greater than the current base interval reduced by a threshold.

10. A pacemaker according to Claim 3, **characterised in that** it prolongs the current base interval by a programmable increment after each rest phase.

11. A pacemaker according to Claim 10, **characterised in that** it prolongs the current base interval by a programmable increment until the minimum base frequency is reached.

12. A pacemaker according to Claim 4, **characterised in that** it reduces the current base interval by a programmable decrement after each activity resumption phase.

13. A pacemaker according to Claim 12, **characterised in that** it reduces the current base interval by a programmable decrement until the programmed base frequency is reached.

14. A pacemaker according to Claim 2, **characterised in that** it defines an excessive extrasystole when, during N cardiac cycles, the number of atrial or ventricular extrasystoles is greater than a programmable value M.

15. A pacemaker according to Claim 14, **characterised in that** it reduces the current base interval by a decrement when it has defined an excessive extrasystole.

16. A pacemaker according to Claim 4, **characterised in that** it defines a sensor activity phase when, less than r times out of p, with r ≤p, the sensor average is less than the rest level of the sensor.

17. A pacemaker according to Claim 4, **characterised in that** it defines a cardiac activity phase when, during N cardiac cycles, the percentage of cardiac cycles for which the interval between two consecutive cardiac events of the same nature is greater than the current base interval reduced by a threshold is less than or equal to a programmable value Y%.

18. A pacemaker according to Claim 4, **characterised in that** it defines a cardiac activity phase when, during S cardiac cycles, the interval between two consecutive cardiac events of the same nature is less than or equal to the current base interval reduced by a threshold.

19. A pacemaker according to Claim 1, **characterised in that** the slaving of the pacing rate to at least one sensor remains always functional whatever the current base frequency.
